**Europäisches Patentamt**

**European Patent Office**

**Office européen des brevets**

(11) Veröffentlichungsnummer: **0 066 822**
A1

# (12) EUROPÄISCHE PATENTANMELDUNG

(21) Anmeldenummer: **82104755.2**

(22) Anmeldetag: **29.05.82**

(51) Int. Cl.³: **B 01 J 10/00, C 12 M 1/08**

(30) Priorität: **06.06.81 DE 3122561**

(43) Veröffentlichungstag der Anmeldung: **15.12.82**
**Patentblatt 82/50**

(84) Benannte Vertragsstaaten: **AT BE CH DE FR GB IT LI NL SE**

(71) Anmelder: **HOECHST AKTIENGESELLSCHAFT, Postfach 80 03 20, D-6230 Frankfurt/Main 80 (DE)**

(72) Erfinder: **Hofer, Norbert, Waldallee 71, D-6239 Eppstein/Taunus (DE)**
Erfinder: **Sittig, Wolfgang, Sulzbacher Weg 2, D-6238 Hofheim am Taunus (DE)**

(54) Verfahren zur Verbesserung der Reaktionsführung in Mammutschlaufenreaktoren.

(57) Zur Verbesserung der Reaktionsführung in Mammutschlaufenreaktoren wird die Geschwindigkeit des umlaufenden Gas-Flüssigkeitsgemisches geregelt. Durch Änderung des Druckverlustes des Gemisches wird die Geschwindigkeit des umlaufenden Gas-Flüssigkeitsgemisches auf 5–100% der maximalen Umlaufgeschwindigkeit eingestellt.

EP 0 066 822 A1

ACTORUM AG

0066822

HOECHST AKTIENGESELLSCHAFT    HOE 81/F 138    D.Ph.HS/sch

Verfahren zur Verbesserung der Reaktionsführung in Mammutschlaufenreaktoren

Gegenstand der Erfindung ist ein Verfahren zur Verbesserung
der Reaktionsführung in flüssigen, begasten Reaktionsgemischen, insbesondere in Fermentationsbrühen und katalysatorhaltigen Gas-Flüssigkeitssystemen in Mammutschlaufenreaktoren durch Regelung der Geschwindigkeit des umlaufenden Gas-Flüssigkeits-Gemisches.

Zur Durchführung solcher Reaktionen ist es erforderlich,
das Gemisch in intensiver Mischbewegung zu halten und den
Stofftransport für die Zuführung der Reaktionspartner und
zur Entfernung der Reaktionsprodukte sicherzustellen.
Gegebenenfalls muß die Geschwindigkeit des Gemisches an
den Wärmeübertragungsflächen hoch genug sein, um bei exothermen Reaktionen die Wärmeabfuhr, bei endothermen Reaktionen die Zufuhr zu gewährleisten.

Es ist bekannt, solche Reaktionen in Rührkesseln, Blasensäulen, Strömungsrohren und Umlaufreaktoren durchzuführen.
Bei Reaktionen mit hohem Gasbedarf eignen sich insbesondere
Schlaufenreaktoren mit Aufstrom- und Abstromteil, deren
Flüssigkeitsantrieb nur durch den Auftrieb eines Gases
sichergestellt wird, wobei im Auftriebsteil die scheinbare Dichte des Gemisches geringer sein muß als im abströmenden Reaktorteil. Die daraus folgende antreibende
Druckdifferenz wird jedoch durch die Verwirbelung an den
Umlenkstellen und durch Reibung des umlaufenden Gemisches
an den Wänden des Auf- und Abstromteiles zu einem beachtlichen Teil aufgezehrt.
Der sich in beiden Reaktorteilen einstellende Gasgehalt
ist u.a. abhängig von den durch die Reaktionsprodukte beeinflußten physikalischen Eigenschaften des Reaktionsgemisches
z.B. der Kulturbrühe (bei Fermentationen), denn Koaleszenzverhalten, Viskosität, Oberflächenspannung und Salzgehalt beeinflussen die Blasenaufstiegsgeschwindigkeit und den Abscheidegrad im Reaktoroberteil. Als Folge hiervon ändert sich die Konzentration der aufsteigenden Gase durch die rückgeführten Gasmengen und die Mischgüte des Systems. Besonders nachteilig ist, wenn die zur Aufrechterhaltung der Mischgüte erforderliche Gasmenge größer sein muß als die für die Reaktion benötigte. Es ist bekannt, Reaktoren so auszulegen, daß für einen optimalen Betriebspunkt die Umlaufrate und das Abscheideverhalten in der Entgasungszone möglichst genau den Anforderungen der Reaktion entsprechen. Bei Abweichen von diesen Auslegungsbedingungen müssen Verschlechterungen von Produktivität und Ausbeute in Kauf genommen werden.

Die Aufgabe zu vorliegender Erfindung besteht demnach darin, unter Beibehaltung des Mammutschlaufenantriebs eine von dem Gaseintrag unabhängige Regelung der Mischgüte, des Abscheideverhaltens und eine Steuerung derselben zu ermöglichen.

Die Aufgabe wird durch ein Verfahren der eingangs genannten Art gelöst, das dadurch gekennzeichnet ist, daß durch Änderung des Druckverlustes des Gemisches die Geschwindigkeit des umlaufenden Gas-Flüssigkeitsgemisches auf 5 bis 100 % der maximalen Umlaufgeschwindigkeit eingestellt wird.

Der Druckverlust kann durch in den Abstromteil oder Aufstiegsteil des Mammutschlaufenreaktors eingebaute Drosseleinrichtungen wie z.B. Wärmetauscher, Gasdispergatoren, Umlenkgitter, verstellbare Ventile und dergleichen erzeugt werden. Der Druckverlust kann in Abhängigkeit vom Gasgehalt im Aufstiegsteil des Mammutschlaufenreaktors eingestellt werden.

Als besonders vorteilhaft hat es sich erwiesen, den Druckverlust durch den Einbau von Drosseleinrichtungen der genannten Art und durch Veränderung der Überlaufhöhe einzustellen.

- 3 -

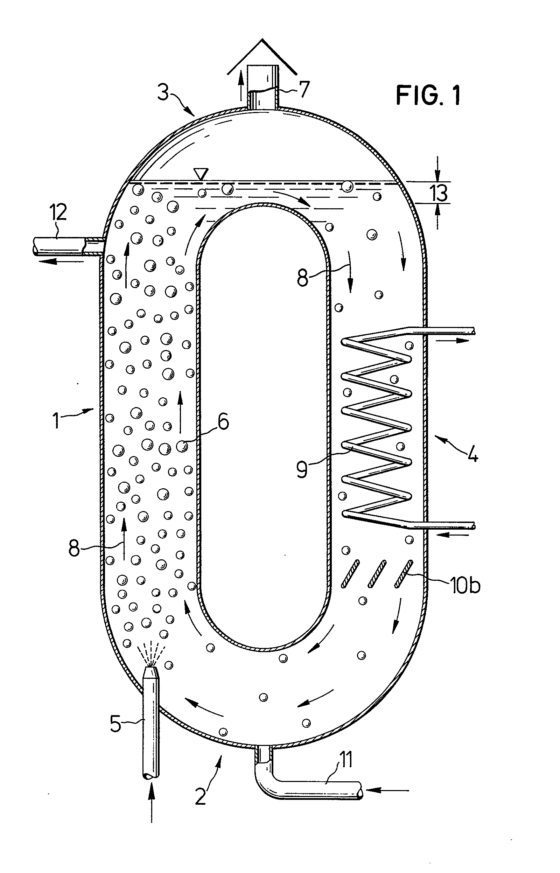

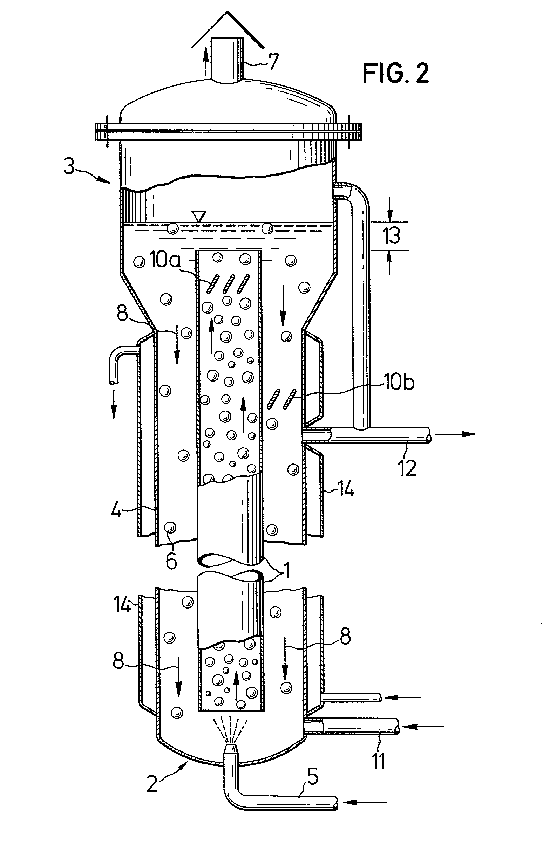

Anhand der Figuren 1 und 2, mit welchen der Verfahrensablauf dargestellt ist, soll das Verfahren näher erläutert werden. In dem aufsteigenden Reaktorteil (1), welcher durch einen unteren Umlaufteil (2) und oberen Umlenkteil (3) mit einem Abstromteil (4) verbunden ist, wird das Antriebsgas, z.B. Reaktionsgas durch eine Begasungsvorrichtung (5) eingetragen. Am oberen Umlenkteil scheidet sich ein Teil des in Blasen (6) dispergierten Gases ab und verläßt durch das Abgasrohr (7) den Reaktor. Durch den Gasgehaltsunterschied in (1) zu dem in (4) wird der durch Strömungspfeile (8) gekennzeichnete Umlauf aufrechterhalten. Wird bei gasverbrauchenden Reaktionen die eingeblasene Gasmenge entsprechend der für die Reaktion benötigten aktuellen Konzentration bemessen, ändert sich entsprechend die Umlauffrequenz des Gemisches und die Abscheidequalität im Kopfteil (3). Durch wärmeabführende Einbauten (9) bzw. Anbauten (14) wird der Umlauf so weit gedrosselt, daß die Mischgüte für die maximal mögliche Reaktionsgeschwindigkeit ausreicht. Durch Schwenken der Klappen (10a und 10b) läßt sich die Umlaufgeschwindigkeit schnell dem Stand der Reaktion anpassen. Durch Regelung der Zuleitung (11) und Ablaufrohr (12) kann die Überlaufhöhe (13) und damit die Umlaufrate längerfristig der Reaktion angepaßt werden.

Beispiel 1

Für die Fermentation von Methylomonas Clara wurde in einen mit Kulturbrühe gefüllten Mammutschlaufenreaktor mit einem Fassungsvermögen von 28m³ 1700 m³ Luft pro Stunde eingetragen und ein Sauerstoffverbrauch von 120 kg/h erzielt. Nach Einbau eines einstellbaren Umlenkgitters betrug der Lufteintrag 1100 m³/h. Der Sauerstoffverbrauch blieb unverändert. Die Ersparnis der Verdichtungskosten betrug somit 35 %. Als Nährmedium wurde eine wäßrige Lösung von 0.15 % $H_3PO_4$, 0.117 % $K_2SO_4$, 0.08 % $MgSO_4 \cdot 7H_2O$, 0.003 % $FeSO_4 \cdot 7H_2O$, 0.024 % $Na_2SO_4$ und 0.0001 % Spurenelemente eingesetzt, die Methanolkonzentration durch ständige Nachgabe auf 10 ppm und der pH-Wert durch Nachdosierung

- 4 -

von NH$_3$-Wasser auf pH 6.8 konstant gehalten. Die Kultivierungstemperatur betrug 38°C, die Zelldichte 15 g/l die Verdünnungsrate 0.25/h.

Beispiel 2

Bei der Herstellung von Acetobacter in einen 2,8 m³ fassenden Mammutschlaufenreaktor wurde die Kulturbrühe in bekannter Weise über eine Ringspaltdüse belüftet. Als Nährmedium wurde Rotwein mit 120 g/m³ Malzextrakt mit 80 g/m³ Ammoniumphosphat eingesetzt. Die Fermentationstemperatur betrug 33°C.

Der Reaktor enthielt Einbauten zum Erzeugen eines Druckverlustes gemäß Erfindung. Es wurde eine Ausbeute an Essigsäure von 92 %, bezogen auf den eingesetzten Alkohol, erzielt. Die Produktivität betrug 1,02 kg/m³·h.

Ohne Einbauten betrug die Ausbeute 90 % und die Produktivität 0.92 kg/m³·h.

Patentansprüche

1. Verfahren zur Verbesserung der Reaktionsführung in flüssigen begasten Reaktionsgemischen in Mammutschlaufenreaktoren durch Regelung der Geschwindigkeit des umlaufenden Gas-Flüssigkeitsgemisches, dadurch gekennzeichnet, daß durch Änderung des Druckverlustes des Gemisches die Geschwindigkeit des umlaufenden Gas-Flüssigkeitsgemisches auf 5 - 100 % der maximalen Umlaufgeschwindigkeit eingestellt wird.

2. Verfahren nach Anspruch 1, dadurch gekennzeichnet, daß der Druckverlust durch Wärmetauscher erzeugt wird.

3. Verfahren nach Anspruch 1, dadurch gekennzeichnet, daß der Druckverlust durch Gasdispergatoren erzeugt wird.

4. Verfahren nach Anspruch 1, dadurch gekennzeichnet, daß der Druckverlust durch verstellbare Umlenkgitter erzeugt wird.

5. Verfahren nach Anspruch 1, 2, 3 oder 4, dadurch gekennzeichnet, daß der Druckverlust in Abhängigkeit vom Gasgehalt im Aufstiegsteil des Mammutschlaufenreaktors eingestellt wird.

0066822

FIG. 1

FIG. 2

# 0066822

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-3 852 384 (J.E.BEARDEN) *Zusammenfassung; Abbildungen 1-5; Spalte 1, Zeilen 3-10; Spalte 2, Zeilen 1-43; Spalte 3, Zeilen 23-31; Spalte 3, Zeile 66 - Spalte 4, Zeile 23* | 1 | B 01 J 10/00 C 12 M 1/08 |
| X | DE-B-1 173 435 (FARBWERKE HOECHST) *Spalte 1, Zeilen 1-4; Spalte 2, Zeile 31 - Spalte 4, Zeile 30; Abbildungen 1-3* | 1,2 | |
| A | GB-A-1 074 932 (THE GAS COUNCIL) *Seite 5, Zeilen 4-8; Abbildung 3* | 4 | |
| A | FR-A-1 602 670 (MAZOWIECKIE ZAKLADY RAFINERYJNE I PETROCHEMICZNE) | 1 | RECHERCHIERTE SACHGEBIETE (Int. Cl. ³) |
| A | DE-C-1 287 035 (EBARA INFILCO) | 1 | B 01 J C 02 F C 12 M |
| A | BE-A- 834 949 (THE BRITISH PETROLEUM) | 1 | |
| A | FR-A-1 222 291 (PATENTAUSWERTUNG VOGELBUSCH) *Seite 5, Spalte 2, Zeilen 1-20; Abbildungen 1-8* | 1 | |

Der vorliegende Recherchenbericht wurde für alle Patentansprüche erstellt.

| Recherchenort | Abschlußdatum der Recherche | Prüfer |
|---|---|---|
| DEN HAAG | 03-09-1982 | SIEM T.D. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82